# EUROPÄISCHE PATENTSCHRIFT

(11) **EP 1 248 775 B1**
(45) Veröffentlichungstag und Bekanntmachung des Hinweises auf die Patenterteilung: **17.03.2004**
(21) Anmeldenummer: 01902309.2
(22) Anmeldetag: 12.01.2001
(51) Int. Cl.: C07D 303/48, C07C 69/732

(54) **VERFAHREN ZUR ENANTIOSELEKTIVEN HERSTELLUNG VON 3,3-DIPHENYL-2,3-EPOXYPROPIONSÄUREESTERN**
METHOD FOR THE ENANTIOSELECTIVE PREPARATION OF 3,3-DIPHENYL-2,3-EPOXY PROPIONIC ACID ESTERS
PROCEDE DE PREPARATION ENANTIOSELECTIVE D'ESTERS D'ACIDE 3,3-DIPHENYL-2,3-EPOXYPROPIONIQUE

(30) Priorität: 20.01.2000 DE 10002393
(43) Veröffentlichungstag der Anmeldung: 16.10.2002
(73) Patentinhaber: Abbott GmbH & Co. KG, 65205 Wiesbaden (DE)
(72) Erfinder: JANSEN, Rolf, 68159 Mannheim (DE)
(74) Vertreter: Grünecker, Kinkeldey, Stockmair & Schwanhäusser Anwaltssozietät
(86) Internationale Anmeldenummer: PCT/EP2001/000322
(87) Internationale Veröffentlichungsnummer: WO 2001/053281

(56) Entgegenhaltungen:
- WO-A-96/11914
- DENIS J.N. ET AL.: "An improved synthesis of the taxol side chain and of RP 56976" JOURNAL OF ORGANIC CHEMISTRY., Bd. 55, Nr. 6, - 16. März 1990 (1990-03-16) Seiten 1957-1959, XP002164880 AMERICAN CHEMICAL SOCIETY. EASTON., US ISSN: 0022-3263
- MUKAIYAMA T. ET AL.: "Reductive cross coupling reaction of a glyoxylate with carbonyl compounds." CHEMISTRY LETTERS., Nr. 8, 1989, Seiten 1401-1404, XP002164881 CHEMICAL SOCIETY OF JAPAN. TOKYO., JP ISSN: 0366-7022

## Beschreibung

Die vorliegende Erfindung betrifft ein Verfahren zur Transformation von 3-Phenylzimtsäureestern in enantiomerenreine 3,3-Diphenyl-2,3-epoxypropionsäureester.

3,3-Diphenyl-2,3-epoxypropionsäureester sind wichtige Zwischenprodukte für die Herstellung von biologisch aktiven Wirkstoffen, wie sie beispielsweise in den Patentanmeldungen WO 96/11914, WO 97/38981, DE 1963046.3 und DE 19944049.2 beschrieben sind. Dort wird die Herstellung dieser Wirkstoffe über racemische und enantiomerenreine Glycidester beschrieben. Im racemischen Verfahren wird der Glycidester über eine Darzen-Glycidestersynthese bereitgestellt. Das racemische Verfahren weist nun den Nachteil auf, dass auf einer späteren Stufe das nicht-gewünschte Enantiomer abgetrennt werden muss, womit ein Verlust in der Gesamtausbeute verbunden ist.

In den beschriebenen Verfahren zur enantiomerenreinen Herstellung der Glycidester geht man dabei von den 3-Phenylzimtsäureestern aus, die durch die Jacobsen-Epoxidierung transformiert werden. Diese Variante weist aber den Nachteil auf, daß die erzielten Enantiomerenüberschüsse nur bei ca. 85 % e.e. liegen und die Aufreinigung der Produkte sehr aufwendig ist. Für ein im technischen Maßstab brauchbares Herstellverfahren sind daher diese bekannten Verfahren nur beschränkt anwendbar.

Eine Dihydroxylierung von Zimtsäuremethylester, Tosylierung der Hydroxyfunktion in 2-Position und anschließende Überführung in das entsprechende Epoxid wird in J. Org. Chem., Vol. 55, No. 6, 1990 offenbart.

Aufgabe der vorliegenden Erfindung ist es, ein Verfahren zur Herstellung von 3,3-Diphenyl-2,3-epoxypropionsäureester bereitzustellen, das eine hohe Enantiomerenreinheit gewährleistet und im technisch-industriellen Maßstab einsetzbar ist.

Diese Aufgabe wird gelöst durch ein Verfahren zur enantioselektiven Herstellung des Glycidesters (I) durch
(a) Dihydroxylierung eines entsprechenden 3-Phenylzimtsäureesters (II) durch Osmium(VIII)oxid-Katalyse in Gegenwart eines Sharpless-Liganden und eines Oxidationsmittels zum Dihydroxyester (III),
(b) selektive Überführung der Hydroxyfunktion in 2-Position des Dihydroxyesters (III) in eine Abgangsgruppe,
(c) intramolekulare Substitution der Abgangsgruppe durch die Hydroxyfunktion in der 3-Position zum Glycidester (I), 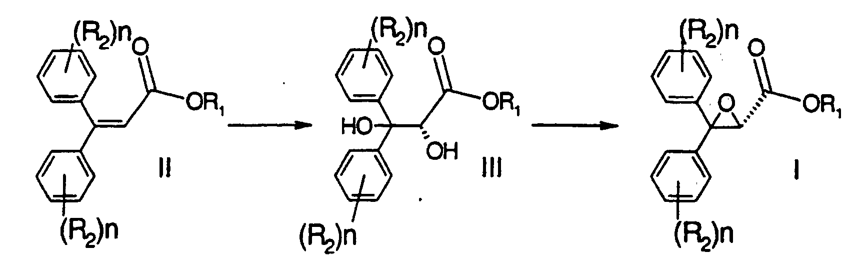
wobei
- R¹: C₁-C₁₀-Alkyl, Aryl oder Arylalkyl, das gegebenenfalls substituiert sein kann, und
- R²: C₁-C₁₀-Alkyl, Aryl, Arylalkyl, Halogen, C₁-C₁₀-Alkoxy, Hydroxy, Acyloxy, Amid oder Amin, das gegebenenfalls substituiert sein kann,
- n: 0 bis 5 bedeutet.

Der Reaktionsschritt (a) ist eine asymmetrische Dihydroxylierung wie sie beispielsweise von Sharpless beschrieben wurde. Die Sharpless-Dihydroxilierung und ihre Anwendung wurde von Kolb, H.C., VanNiewenhze, M.S., und Sharpless, K.B., Chem. Rev. 1994, 94, 2483 in einem Review ausführlich beschrieben, auf dessen Inhalt hiermit ausdrücklich Bezug genommen wird. In einem Beitrag von L. Ahrgren und L. Sutin in Organic Process Research& Development 1997, 1, 425-427 wurde gezeigt, dass die Reaktion prinzipiell auch in größerem Maßstab (upscale-bar) sein sollte.

Unter Osmium(VIII)oxid-Katalysator sind Sauerstoffverbindungen von Osmium mit der Oxidationsstufe + VIII zu verstehen, bevorzugt sind OsO₄, und K₂OsO₂(OH)₄.

Das Osmium(VIII)oxid braucht dabei nicht in stöchiometrischen Mengen zum Substrat der Formel (II) eingesetzt werden, sondern kann als Katalysator in sehr viel geringeren Mengen eingesetzt werden, wobei der verbrauchte Katalysator durch ein Oxidationsmittel regeneriert wird.

Der Osmium(VIII)oxid-Katalysator wird üblicherweise in Mengen von 0,1 bis 5, bevorzugt 0,1 bis 2,5 Mol %, bezogen auf das Substrat der Formel (II) eingesetzt.

Als Sharpless Liganden werden solche Verbindungen bezeichnet, die chiral sind, einen Stickstoff-haltigen sechsgliedrigen Heterozyklus besitzen, an OsO₄ binden und die OsO₄-bedingte Dihydroxylierung beschleunigen.

Solche Sharpless-Liganden sind bevorzugt Hydrochinin- und Hydrochinidinderivate, die beispielsweise aus der oben angegebenen Literatur bekannt sind. Bevorzugt sind Hydrochinin-(1,4-phthalazindiyldiether) (DHQ)2PHAL bzw. Hydrochinidin-(1,4-phthalazindiyldiether) (DHQD)2PHAL bzw. Hydrochinin-(2,5-diphenyl-4,6-pyrimidindiyldiether) (DHQ)2PYR bzw. Hydrochinidin-(2,5-diphenyl-4,6-pyrimidindiyldiether) (DHQD)2PYR.

Die Wahl des Sharpless Liganden richtet sich natürlich nach dem gewünschten Enantiomer (III). Je nachdem von welcher Seite der Angriff auf die zu hydroxylierende Doppelbindung von (II) erfolgen soll, muß nach den bekannten mechanistischen Regeln von Sharpless ein entsprechender Sharpless Ligand ausgewählt werden.

Für den Schritt (a) geeignete Oxidationsmittel sind solche Stoffe, die in der Lage sind, das bei der Dihydroxylierung der Doppelbindung reduzierte Osmiumoxid wieder in die aktive Oxidationsstufe + VIII zu überführen, d.h. die Reaktivierung des Dihydroxylierungskatalysators erlauben. Solche Oxidationsmittel können leicht aufgrund ihres elektrochemisches Potentials ermittelt werden. Gut geeignete Oxidationsmittel sind beispielsweise Sauerstoff, Wasserstoffperoxid, N-Methylmorpholin-N-oxid (NMO), Kaliumhexacyanoferrat, der AD-Mix nach Sharpless (J. Org. Chem., 1992, 57, 2768) oder auch elektochemische Verfahren.

Weitere Oxidationsmittel werden beispielsweise von M. Schröder, Chem. Rev. 1980, 80, 187-213 beschrieben.

Bevorzugt werden Sauerstoff, Wasserstoffperoxid, N-Methylmorpholin-N-oxid, Kaliumhexacyanoferrat und elektrochemische Verfahren als Oxidationsmittel verwendet.

Für die technische Umsetzung gemäß (a) hat sich als besonders geeignet erwiesen eine Mischung von 0,1 bis 1,5 Molprozent K2OsO2(OH)₄, 0,1 bis 3 Molprozent Hydrochinin-(1,4-phthalazindiyldiether) (DHQ)2PHAL bzw. Hydrochinidin-(1,4-phthalazindiyldiether) (DHQD)2PHAL bzw. Hydrochinin-(2,5-diphenyl-4,6-pyrimidindiyldiether) (DHQ)2PYR bzw. Hydrochinidin-(2,5-diphenyl-4,6-pyrimidindiyldiether) (DHQD)2PYR und 100 bis 300 Molprozent NMO, jeweils bezogen auf die Menge an 3-Phenylzimtsäureester (II).

Als geeignete Lösungsmittel werden Gemische von Wasser mit einem organischen Lösungsmittel verwendet.

Als besonders geeignet erwiesen haben sich Mischungen von Wasser mit Alkoholen, Acetonitril und Aceton im Verhältnis 0,1 bis 5 zu 1 (Gewichtsverhältnis).

Die Reaktionstemperatur liegt im Bereich von -20°C bis 100°C, bevorzugt werden Temperaturen kleiner 75°C.

Bevorzugt wird zur Umwandlung des Diols in ein Epoxid das Diol mit 1 bis 10 Äquivalenten einer Base und 1 bis 3 Äquivalenten eines Sulfonsäurechlorids, eines Azodicarbonsäurederivats oder eines Carbodiimids versetzt. Insbesondere werden als Basen tertiäre Amine und zur Generierung einer Abgangsgruppe Sulfonsäurechloride bevorzugt.

Zur Einführung der Abgangsgruppe im Reaktionsschritt (b) werden bevorzugt verwendet Sulfonsäurechloride wie Methansulfonsäurechlorid und Toluolsulfonsäurechlorid.

Die Reaktion kann als sowohl als Eintopfverfahren ohne Isolierung einer Zwischenstufe als auch zweistufig mit Isolierung der Vorstufe des Epoxids durchgeführt werden. Eine bevorzugte Ausführungsform ist das Eintopfverfahren.

Als Lösungsmittel für (b) bzw (c) werden aprotische Lösungsmittel mittlerer Polarität wie z.B. Toluol oder Methylenchlorid bevorzugt.

Die Reaktion läuft bei Temperaturen von -70°C bis 100°C ab. Bevorzugt sind Reaktionstemperaturen zwischen -10°C bis 35°C.

Die Herstellung der entsprechenden 3-Phenylzimtsäureester (II) ist dem Fachmann aus Standardwerken der organischen Literatur bekannt.

Die Überführung von 1,2-Diolen in Epoxide ist allgemein bekannt und wird z.B. im Lehrbuch J. March Advanced Organic Chemistry, 1985, bei Wiley auf den Seiten 270 bzw 345 beschrieben.

Die Erfindung wird durch die folgenden Beispiele weiter erläutert.

### Allgemeine Arbeitsvorschrift:

1.) Synthese der 2,3-Dihydroxy-3,3-diphenylpropionsäureester
   a) AD-Mix Variante:
      In einem Kolben wurden 27,2 g AD-Mix-alpha vorgelegt, 100 ml Wasser zugegeben, 80 ml tert-Butanol zugegeben und zu diesem Gemisch 5,6 g 3-Phenylzimtsäure-tert.-butylester gelöst in 20 ml tert.-Butanol Die Reaktion war bei Raumtemperatur nach 3 Tagen vollständig. Zu dem Zweiphasensystem wurden 30 g Natriumsulfit gelöst in Wasser gegeben und eine halbe Stunde gerührt. Die organische Phase wurde abgetrennt, die wässrige Phase mit Methylenchlorid extrahiert und die vereinigten organischen Phasen nochmals mit Wasser gewaschen. Anschließend wurde über Magnesiumsulfat getrocknet und das Lösungsmittel abdestilliert. Es wurden 4,8 g eines flockigen Feststoffs isoliert (HPLC-Reinheit 95 %, das falsche Enantiomere wurde nicht beobachtet).
   b) N-Methyl-Morpholin-N-Oxid Variante (langsame Substratzugabe):
      In einem Kolben wurden 250 mg K₂OsO₂(OH)₄, 750 mg des Hydroquinidine 1,4 phthalazinediylethylether und 30 ml 50 %ige N-Methyl-Morpholin-N-Oxid-Lösung in 120 ml tert-BuOH und 90 ml Wasser vorgelegt und solange gerührt bis alles gelöst war. 24 g des 3-Phenylzimtsäureethylesters wurden mit tert.-Butanol auf ein Volumen von 48 ml aufgefüllt und mit einer Geschwindigkeit von 1 ml/h direkt in die Lösung geleitet. Nach ca 1 Stunde verfärbte sich die Lösung von Violett nach Gelb und nach drei Stunden begann ein Feststoff auszufallen. Nach 28 Stunden hatte sich ein fester Brei gebildet und nach 50 Stunden wurde in dem Natriumsulfit-Lösung zugegeben wurde und 30 Minuten nachgerührt wurde. Anschließend wurde Methylenchlorid und 1 N HCl zugegeben, die organische Phase abgetrennt und das Lösungsmittel abdestilliert. Das Rohprodukt wurde in Heptan umkristallisiert und es wurden 17 g Produkt als weißer flockiger Niederschlag (98 % Reinheit, 61 % Ausbeute) isoliert.
   c) N-Methyl-Morpholin-N-Oxid Variante (direkte Substratzugabe):
      In einem Kolben wurden 30 mg K₂OsO₂(OH)₄, 95 mg des (DHQ)2PHAL und 4,2 ml 50 %ige N-Methyl-Morpholin-N-Oxid-Lösung in 90 ml Isopropanol und 30 ml Wasser vorgelegt und 2 Stunden gerührt. Zu der Lösung wurden 10 g des 3-Phenylzimtsäureethylesters gegeben und 48 Stunden bei 0°C gerührt. Zur Aufarbeitung wurde Natriumsulfit-Lösung zugegeben und 30 Minuten nachgerührt. Anschließend wurden 50 ml Methylenchlorid und 50 ml 1 N HCl zugegeben, die organische Phase abgetrennt und das Lösungsmittel abdestilliert. Das Rohprodukt wurde in MTB/Heptan umkristallisiert und es wurden 7 g Produkt als weißer flockiger Niederschlag isoliert.
   R-2,3-Dihydroxy-3,3-diphenylpropionsäureethylester [alpha] 20 = - 149 (Methanol, c = 1,589 nm).
2.) Bildung des Glycidesters
   Der 2,3-Dihydroxy-3,3-diphenylpropionsäureethylester (12,4 g, 43 mmol) wurde in 260 ml CH₂Cl₂ vorgelegt und bei 0°C erst Triethylamin (17,5 g, 101 mmol) und dann Mesylchlorid (10 g, 86 mmol) zugegeben. Die Lösung wurde 48 Stunden bei Raumtemperatur gerührt. Zur Aufarbeitung wurde die Methylenchloridphase mit 100 ml HCl 1 N und mit NaHCO₃ Lösung gewaschen. Nach azeotroper Destillation von Wasserresten mit Methylenchlorid kann der Glycidester in Lösung weiter umgesetzt werden. Zur Ausbeutebestimmung wurde das Lösungsmittel abdestilliert und es wurden 13 g eines Öls isoliert, welches laut HPLC eine Reinheit von 94 % aufwies.

## Patentansprüche

1. Verfahren zur enantioselektiven Herstellung des Glycidesters (I) durch
(a) Dihydroxylierung eines entsprechenden 3-Phenylzimtsäureesters (II) durch Osmium(VIII)oxid-Katalyse in Gegenwart eines Sharpless-Liganden und eines Oxidationsmittels zum Dihydroxyester (III),
(b) selektive Überführung der Hydroxyfunktion in 2-Position des Dihydroxyesters (III) in eine Abgangsgruppe,
(c) intramolekulare Substitution der Abgangsgruppe durch die Hydroxyfunktion in der 3-Position zum Glycidester (I), 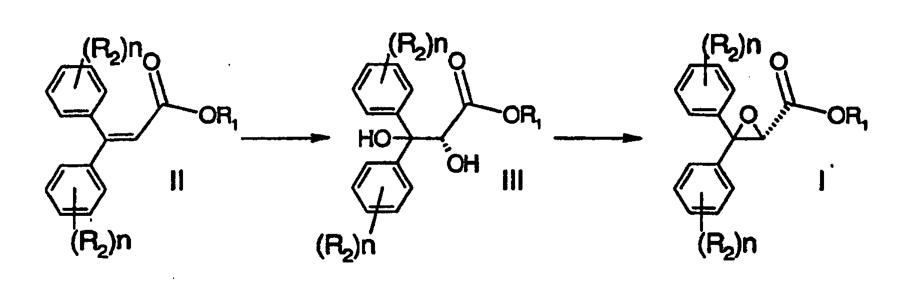
wobei
R₁ C₁-C₁₀-Alkyl, Aryl oder Arylalkyl, das gegebenenfalls substituiert sein kann,
R₂ C₁-C₁₀-Alkyl, Aryl, Arylalkyl, Halogen, C₁-C₁₀-Alkoxy, Acyloxy oder Amid, das gegebenenfalls substituiert sein kann, und
n 0 bis 5 bedeutet.

2. Verfahren nach Anspruch 1, **dadurch gekennzeichnet, dass** die Transformation von Dihydroxyester (III) zu Glycidester (I) in einem Eintopfverfahren ohne Isolierung der Zwischenstufe durchgeführt wird.

3. Verfahren nach Anspruch 1, **dadurch gekennzeichnet, dass** als Verbindung (III) Zimtsäurederivate mit R¹ = Alkyl, Benzyl und R² = H eingesetzt werden.

## Claims

1. Process for enantio-selective production of glycidyl. ester (I) by
(a) dihydroxylation of a corresponding 3-phenyl cinnamic acid ester (II) with osmium(VIII)oxide catalysis in the presence of a Sharptess ligand and an oxidising agent to dihydroxyester (III),
(b) selective conversion of the hydroxy function in 2-position of dihydroxy ester (III) to a leaving group,
(c) intramolecular substitution of the leaving group by the hydroxy function in 3-position to glycidyl ester (I), 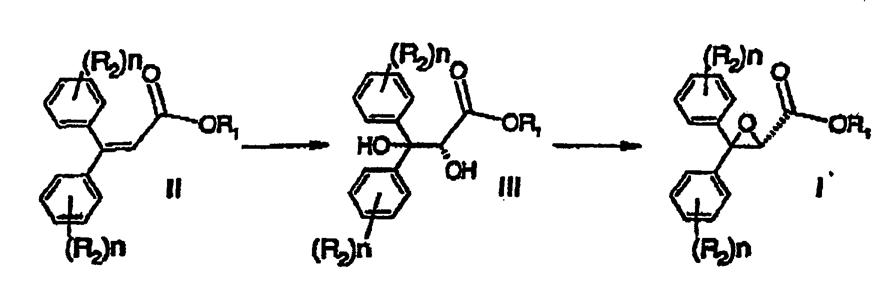
wherein
R₁ is C₁-C₁₀ alkyl, aryl or arylalkyl, which can optionally be substituted,
R₂ is C₁-C₁₀ alkyl, aryl, arylalkyl, halogen, C₁-C₁₀-alkoxy, acyloxy or amide which can optionally be substituted, and
n means 0 - 5.

2. Process according to claim 1, **characterised in that** the transformation of dihydroxyester (III) to glycidyl ester (I) is carried out in a one-pot process without isolating the intermediate stage.

3. Process according to claim 1, **characterised in that** cinnamic acid derivatives in which R₁=alkyl, benzyl and R₂=H are used as compound (III).

## Revendications

1. Procédé de préparation énantiosélective du glycidester (I) par
(a) dihydroxylation en le dihydroxyester (III) d'un ester d'acide 3-phénylcinnamique (II) correspondant par catalyse avec l'oxyde d'osmium (VIII) en présence d'un ligand de Sharpless et d'un oxydant,
(b) conversion sélective de la fonction hydroxyle en position 2 du dihydroxyester (III) en un groupe partant,
(c) substitution intramoléculaire du groupe partant par la fonction hydroxyle en position 3 pour former le glycidester (I) 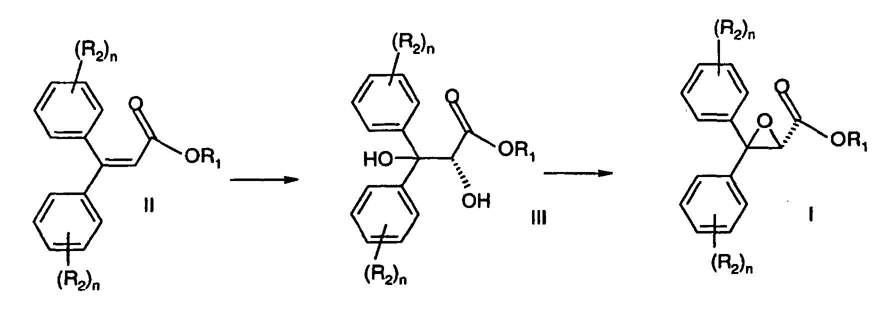
où
R₁ représente alkyle en C₁-C₁₀, aryle ou arylalkyle, qui peut éventuellement être substitué,
R₂ représente alkyle en C₁-C₁₀, aryle, arylalkyle, halogène, alcoxy en C₁-C₁₀, acyloxy ou amide, qui peut éventuellement être substitué, et
n représente 0 à 5.

2. Procédé selon la revendication 1 **caractérisé en ce que** la transformation du dihydroxyester (III) en le glycidester (I) est réalisée dans un procédé à un seul récipient sans isolement de l'intermédiaire.

3. Procédé selon la revendication 1 **caractérisé en ce que** des dérivés de l'acide cinnamique avec R¹ = alkyle, benzyle et R² = H sont utilisés comme composé (III).
